# EUROPEAN PATENT APPLICATION

(11) **EP 1 719 518 A1**
(43) Date of publication of application: **08.11.2006**
(21) Application number: 06075064.3
(22) Date of filing: 06.08.1999
(51) Int. Cl.: A61K 35/74, A61K 45/06, A61P 3/02, A23L 1/30, A61K 31/715, A61K 31/00, A61K 33/00, A61K 31/70

(54) **Compositions comprising bacillus coagulans for increasing the solubility and bioavailability of nutritional minerals**

(30) Priority: 07.08.1998 US 95786 P; 05.08.1999 US 369016
(62) Divisional of application: 99945016.6
(71) Applicant: Ganeden Biotech, Inc., San Diego, CA 92121 (US)
(72) Inventor: Farmer, Sean, Miami Beach, FL 33140 (US)
(74) Representative: Crump, Julian Richard John

(57) **Abstract**

A non-therapeutic use of one or more non-pathogenic, lactic acid-producing strains of *Bacillus coagulans,* or of an extracellular supernatant derived from a culture of one or more such strains, within a nutritionally-acceptable carrier as a nutritional composition for increasing the solubility and bioavailability of nutritional minerals within the gastrointestinal tract of the animal. The one or more strains of *Bacillus coagulans* may be selected from *Bacillus coagulans, Bacillus coagulans Hammer, Bacillus brevis* subspecies *coagulans* and any genetic variants thereof. The nutritional minerals may be present in the form of gluconates or citrates.

## Description

### FIELD OF THE INVENTION

The present invention relates to the utilization of probiotic lactic acid-producing bacteria in a nutritional composition. More specifically, the present invention relates to the use of *Bacillus cocigulans* for increasing the solubility of nutritional materials, preferably essential vitamins and minerals, within the gastrointestinal tract of mammals.

### BACKGROUND OF THE INVENTION

### 1. Probiotic Microorganisms

Probiotic microorganisms are those which confer a benefit when grow in a particular environment, often by inhibiting the growth of other biological organisms in the same environment. Examples of probiotic organisms include bacteria and bacteriophages which possess the ability to grow within the gastrointestinal tract, at least temporarily, to displace or destroy pathogenic organisms, as well as providing other benefits to the host. See *e.g.*, Salminen *et al,* 1996. *Antonie Van Leeuwenhoek 70:* 347-358; Elmer *et al,* 1996. *JAMA* 275: 870-876; Rafter. 1995. *Scond. J. Gastroenterol. 30:* 497-502; Perdigon *et al,* 1995. *J. Dairy Sci, 78*: 1597-1606; Gandi, *Townsend Lett, Doctors & Patients,* pp. 108- 110, Jan. 1994; Lidbeck *et al*, 1992. *Eur. J. Cancer Prev. 1:* 341-353.

The majority of previous studies on probiosis have been observational rather than mechanistic in nature, and thus the processes responsible for many probiotic phenomena have yet to be quantitatively elucidated. Some probiotics are members of the normal colonic microflora and are not viewed as being overtly pathogenic. However, these organisms have occasionally caused infections (*e.g.*, bacteremia) in individuals who are, for example, immunocompromised. See *e.g.,* Sussman, J. *et al.,* 1986. *Rev Infect. Dis. 8*: 771-776; Hata, D. *et al.,* 1988. *Pediatr. lnfect. Dis. 7*: 669-671.

For example, the probiotic bacteria found in sour milk, has been utilized since ancient times (*i.e*., long-before the discovery of bacteria) as a therapeutic treatment for dysentery and related gastrointestinal diseases. More recently, probiotic preparations were systematically evaluated for their effect on health and longevity in the earty-1900's (see *e.g.*, Metchinikoff, E., *Prolongation of Life.* Willaim Heinermann. London 1910), although their utilization has been markedly limited since the advent of antibiotics in the 1950's to treat pathological microbes. See *e.g.*, Winberg, *et al.* 1993. *Pediatr. Nephrol. 7*:509-514: Malin *et al. Ann, Nutr. Metab. 40:* 137-145; and U.S. Patent No. 5,176.911. Similarly, lactic acid-producing bacteria (*e.g., Bacillus. Lactobacillus* and *Streptococcus* species) have been utilized as food additives and there have been some claims that they provide nutritional and/or therapeutic value. See *e.g.,* Gorbach. 1990. *Ann. Med. 22*: 37-41; Reid *et al,* 1990. *Clin. Microbiol, Rev. 3*: 335-344.

The best known probiotics are the lactic acid-producing bacteria (*i.e., Lactobacilli*) and *Bifidobacteria,* which are widely utilized in yogurts and other dairy products. These probiotic organisms are non-pathogenic and non-toxigenic, retain viability during storage, and possess the ability to survive passage through the stomach and small intestine. Since probiotics do not petmanently colonize the host, they need to be ingested or applied regularly for any health-promoting properties to persist. Commercial probiotic preparations are generally comprised of mixtures of *Lactobacilli* and *Bifidobacterio,* although yeast such as *Saccharomyces* have also been utilized.

### 2. Gastrointestinal Microflora

Perhaps the best-charaeterized use of probiotic microorganisms is in the maintenance of gastrointestinal microflora. The gastrointestinal microflora has been shown to play a number of vital roles in maintaining gastrointestinal tract function and overall physiological health. For example, the growth and metabolism of the many individual bacterial species inhabiting the gastrointestinal tract depend primarily upon the substrates available to them, most of which are derived from the diet. See *e.g*., Gibson G.R. *et al.,* 1995. *Gastroenterology 106:* 975-982; Christl, S.U. *et al.,* 1992. *Gut 33:* 1234-1238. These finding have led to attempts to modify the structure and metabolic activities of the community through diet, primarily with probiotics which are live microbial food supplements.

While the gastrointestinal microflora presents a microbial-based barrier to invading organisms, pathogens often become established when the integrity of the microbiota is impaired through stress, illness, antibiotic treatment, changes in diet, or physiological alterations within the gastrointestinal tract. For example, *Bifidobacteria* are known to be involved in resisting the colonization of pathogens in the large intestine. See *e.g..* Yamazaki, S, *et al.,* 1982. *Bifidobacteria and Microflora l:* 55-60. Similarly, the administration of *Bifidobacteria breve* to children with gastroenteritis eradicated the causative pathogenic bacteria (*i.e., Campylobacter jejuni*) from their stools (see *e.g.,* Tojo. M. 1987. *Acta Pediatr. Jpn. 29*: 160-167) and supplementation of infant formula milk with *Bifidobacteria bifidum* and *Streptococcus thermophilus* was found to reduce rotavirus shedding and episodes of diarrhea in children who were hospitalized (see *e.g.,* Saavedra. J.M., 1994. *The Lancet 344*: 1046-109.

In addition, some lactic acid producing bacteria also produce bacteriocins which are inhibitory metabolites which are responsible for the bacteria's anti-microbial effects. See *e.g.*, Klaenhammer, 1993. *FEMS Microbiol, Rev.* 12: 39-85; Barefoot *et al.,* 1993. *J. Diary Sci. 76:* 2366-2379. For example, selected *Lactobacillus* strains which produce antibiotics have been demonstrated as effective for the treatment of infections, sinusitis, hemorrhoids, dental inflammations, and various other inflammatory conditions. See *e.g.,* U.S. Patent No. 5,439,995. Additionally, *Lactobacillus reuteri* has been shown to produce antibiotics which possess anti-microbial activity against Gram negative and Gram positive bacteria, yeast, and various protozoan. See *e.g.,* U.S. Patent Nos. 5,413,960 and 5,439,678. *Lactobacillus casei ssp, rhamnosus* has been utilized to preserve animal feedstuffs, particularly *S. rubidaea* F 13299, alone or combined with an anti-fungal *B. sublillis* (swain FB260). See U.S. Patent No. 5,371,011.

Probiotics have also been shown to possess anti-mutagenic properties. For example, Gram positive and Gram negative bacteria have been demonstrated to bind mutagenic pyrolysates which are produced during cooking at a high temperature. Studies performed with lactic acid-producing bacteria has shown that these bacteria may be either living or dead, due to the fact that the process occurs by adsorption of mutagenic pyrolysates to the carbohydrate polymers present in the bacterial cell wall. See *e.g.,* Zang, X. *Bacillus et al.,* 1990. *J. Dairy Sci.* 73: 2702-2710. *Lactobacilli* have also been shown to possess the ability to degrade carcinogens (*e.g.*, N-nitrosamines), which may serve an important role if the process is subsequently found to occur at the level of the mucosal surface. See *e.g.,* Rowland, I.R. and Grasso, P., *Appl*. *Microbiol. 29*: 7.12. Additionally, the co-administration of lactulose and *Bifidobacteria longum* to rats injected with the carcinogen azoxymethane was demonstrated to reduce intestinal aberrant crypt foci, which are generally considered to be pre-ncoplastic markers. See *e.g.,* Challa, *A. et al*., 1997. *Carcinogenesis 18:* 5175-21. Purified cell walls of *Bifidobacteria* may also possess anti-tumorigenic activities in that the cell wall of *Bifidobacteria infantis* induces the activation of phagocytes to destroy growing tumor cells. See *e.g.,* Sekine. K. *et al.,* 1994. *Bifidobacteria and Microflora 13:* 65-77. *Rifidobacteria* probioties have also been shown to reduce colon carcinogenesis induced by 1.2-dimethylhydrazine in mice when concomitantly administered with fructo-oligosaccharides(FOS; see *e.g.,* Koo and Rao. 1991. *Nutril. Rev. 51:* 137-146). as well as inhibiting liver and mammary tumors in rats (see *e.g.,* Reddy and Rivenson. 1993. *Cancer Res. 53*: 3914-3918).

### 3. Physiological Effects of Antibiotic Administration

Antibiotics are widely used to control pathogenic microorganisms in both humans and animals. Unfortunately, the widespread use of anti-microbial agents, especially broad spectrum antibiotics, has resulted in a number of serious clinical consequences. For example, the indiscriminate use of these chemicals has resulted in the generation of multiple antibioticresistant pathogens. See *e.g.,* Mitchell, P. 1998. *The Lancet 352:* 462-463; Shannon, K., 1998. *The Lancet 352:* 490-491.

In addition, antibiotics often kill beneficial, non-pathogenic microorganisms (*i.e.,* flora) within the gastrointestinal tract which contribute to digestive function and health. Accordingly, relapse (the return of infections and their associated symptoms) and secondary opportunistic infections often result from the depletion of lactic acid-producing and other beneficial flora within the gastrointestinal tract. Most, if not all, lactic acid-producing or probiotic bacteria are extremely sensitive to common antibiotic compounds. During a normal course of antibiotic therapy, many individuals develop a number of deleterious physiological side-effects including: diarrhea, intestinal cramping, and sometimes constipation. These side-effects are primarily due to the non-selective action of antibiotics, as antibiotics do not possess the ability to discriminate between beneficial, non-pathogenic and pathogenic bacteria, both bacterial types are killed by these agents. Thus, individuals taking antibiotics offer suffer from gastrointestinal problems as a result of the beneficial microorganisms (*i.e.,* intestinal flora), which normally colonize the gastrointestinal tract, being killed or severely attenuated. The resulting change in the composition of the intestinal flora can result in vitamin deficiencies when the vitamin-producing intestinal bacteria are killed, diarrhea and dehydration and, more seriously, illness should a pathogenic organism overgrow and replace the remaining beneficial gastrointestinal bacteria.

In addition to the gastrointestinal microflora, beneficial and/or pathological microorganisms can also inhabit the oral cavity, the genital area and the vagina (see *e.g.,* Thomason, et al, 1991. *Am. J. Obstet Gynecol.* 165: 1210-1217; Marsh. 1993. *Caries Res.* 27: 72-76; Lehner, 1985. *Vaccine* 3: 65-68; Hill & Embil. 1986. Can. *Med. Assoc. J.* 134: 321-331). The use of anti-microbial drugs can similarly cause an imbalance in those microorganisms and the therapeutic use of probiotic bacteria, especially the *Lactobacillus* strains, which colonize those areas has been disclosed (see *e.g.,* Winberg, *et al..* 1993. *Pediatr. Nephrol.* 7: 509-514; Malm. *et al.,* 1996. *Ann. Mar. Metab.* 40: 137-145. U.S. Patent No. 5-176.911), Increasing numbers of pathogenic microorganisms have developed antibiotic resistance, requiring the development and use of second and third generation antibiotics. Microorganisms that arc resistant to multiple drugs have also developed often with multiple drug resistance spreading between species, leading to serious infections that cannot be controlled by use of antibiotics.

### 4. Bacillus coagulans

*Bacillus coagulans* is a non-pathogenic gram positive spore-forming bacteria that produces L(+) lactic acid (dextrorotatory) in homo-fermentation. This microorganism has been isolated from natural sources, such as heat-treated soil samples inoculated into nutrient medium (see *e.g., Bergey's Manual of Systemic Bacteriology,* Vol. 2, Sneatlt, P.H.A., *et al.,* eds., (Williams & Wilkins, Baltimore, MD, 1986)).

Purified *Bacillus coagulans* strains have served as a source of various enzymes including, but not limited to: restriction endonucleases (see U.S. Patent No. 5,200,336); amylase (see U.S. Patent No. 4,980,180); lactase (see U.S: Patent No. 4,323,651); and cyclo-malta-dextrin glucano-transferase (see U.S. Patent No. 5,102,800). *Bacillus coagulans* has been used to produce lactic acid (see U.S. Patent No. 5,079,164). In addition, a strain of *Bacillus coagulans* (designated *Lactobacillus sporogenes,* Sakaguti & Nakayama (ATCC 31284)) has been combined with other lactic acid-producing bacteria and *Bacillus natto* to produce a fermented food product from steamed soybeans (see U.S. Patent No. 4,110,477). *Bacillus coagulans* strains have also been used as animal feed additives for poultry and livestock to reduce disease and improve feed utilization and to therefore, increase growth rate in the animals (see International Patent Application Nos. WO 9314187 and WO 9411492).

In addition, *Bacillus coagulans* strains and various other lactic acid-producing bacteria have proven to produce copious amounts of lactic acid and enzymes that have the ability to increase the solubility of nutritional materials, in particular vitamins and minerals.

Accordingly, there remains an, as yet unfulflled, need for a composition which possesses the ability to increase the solubility and hence, bioavailability of nutritional materials, preferably vitamins and minerals, within the gastrointestinal tract of animals or humans.

### SUMMARY OF THE INVENTION

The present invention discloses the discovery that, in digestion, bacterial enzymes and various other metabolic products of bacteria (*e.g.*, lactic acid), play a vital role in the solubility and therefor the bioavailability, of vitamins and minerals, either as a component of food or as a result of nutritional supplementation.

The present invention discloses a probiotic composition comprising one or more *Bacillus* bacterial species or strains, combined with an effective amount of a bifidogenic oligosaccharide, preferably fructo-oligosaeeharide(FOS), in a pharmaceutically- or nutritionally-acceptable carrier which is suitable for oral administration to a human or animal species, wherein said *Bacillus*: *(i*) is capable of growing at temperatures of about 20°C to about 65°C; (*ii*) produces L(+) lactic acid (dextrorotatory); (*iii*) produces spores resistant to heat up to 98°C; and (*iv*) exhibits probiotic activity. In one embodiment, the probiotic activity results from the vegetative growth of the isolated *Bacillus. Lactobacillus, Bifidlobacterium,* or *Sporolactobocillus* species that produces extracellular metabolites which function to increase the bioavailability of nutrients though increased solubility.

The present invention also discloses a therapeutic composition comprising one or more *Bacillus coagulans* strain in combination with a therapeutically-effective amount of a short or long chain bifidogenic oligosaccharide, preferably fructo-oligosaccheride (FOS), in a pharmaceutically- or nutritionally-acceptable carrier suitable for oral administration to the digestive track of a mammal. In different embodiments of the aforementioned composition, the *Bacillus coagulans* strain is included in the composition in the form of spores which are capable of germinating following ingestion: a dried cell mass; a stabilized gel or paste: or a stabilized liquid suspension..

The present invention also describes a method for decreasing the number of pathogenic organisms that inhibit the growth of vitamin-producing organisms within the gastrointestinal tract, wherein a composition is administered that would, preferably, include a complex of the RDA vitamins, minerals and trace minerals as well as those nutrients that have no established RDA, but have a beneficial role in healthy human or mammal physiology. The preferred composition would include a minerals which are in either in the gluconate or citrate form, due to the established fact that these forms are more readily metabolized by lactic acid-producing bacteria.

It should be understood that both the foregoing general description and the following detailed description are exemplary and explanatory only and are not restrictive of the invention as claimed.

### DESCRIPTION OF THE FIGURES

FIG. 1: illustrates, in tabular form, the various metabolic characteristics of *Bacillus coagulans.*
FIG. 2: illustrates, in tabular form, the vitamins, their RDA, their ranges and respective preferred ranges, of the therapeutic compositions of the present invention.
FIG. 3: illustrates, in tabular form, the preferred major minerals and their respective RDA.
FIG. 4: illustrates, in tabular form, the preferred trace minerals and their respective ranges, as utilized in the therapeutic composition of the present invention.

### DETAILED DESCRIPTION OF THE INVENTION

The disclosures of one or more embodiments of the invention are set forth in the accompanying description below. Although any methods and materials similar or equivalent to those described herein can be used in the practice or testing of the present invention, the preferred methods and materials are now described. Other features, objects, and advantages of the invention will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this invention belongs. Unless expressly stated otherwise, the techniques employed or contemplated herein are standard methodologies well known to one of ordinary skill in the art. The examples of embodiments are for illustration purposes only. All patents and publications cited in this specification are incorporated by reference.

As utilized herein, the term "probiotic" refers to microorganisms (*e.g.*, bacteria, yeast, viruses, and/or fungi) which form, at a minimum, a part of the transient or endogenous flora and, thus, possess a beneficial prophylactic and/or therapeutic effect upon the host organism. Probiotics are generally known to be clinically-safe (*i.e..* non-pathogenic) by those skilled within the art. Although not wishing to be bound by any particular mechanism, the probiotic activity of, for example, the *Bacillus* species, is thought to result from competitive inhibition of growth of pathogens due to superior colonization, parasitism of undesirable microorganisms, lactic acid production and/or other extracellular products having anti-microbial activity, or combinations thereof.

As utilized herein, the term ''nutritional supplement" refers to any type of orally ingestable material which can be digested and assimilated by the gastrointestinal tract, and from which a physiological, nutritional benefit may be derived. This material includes liquid and solid foodstuffs, vitamin and mineral supplements, and the like.

The present invention discloses the utilization of *Bacillus, Lactobacillus, Bifidiobacterium,* or *Sporolactobacillus* bacterial species, preferably *Bacillus coagularts,* in nutritional compositions, in combination with a bifidogenic oligosaccharide, preferably fructo-oligosaccharides (FOS), as a probiotic for increasing the solubility of essential vitamin and mineral nutritional materials in mammals, particularly in humans. The present invention therefore describes various nutritional compositions, methods for using the compositions and systems containing the nutritional compositions.

Non-pathogenic *Bacillus* species of the present invention include, but are not limited to: *Bacillus coagulans; Bacillus coagulans Hammer; Bacillus brevis* subspecies *coagulans, Bacillus subtilis, Bacillus uniflagellatus, Bacillus lateropsorus, Bacillus laterosporus* BOD, *Bacillus megaterium, Bacillus polymyxa, Bacillus licheniformis, Bacillus pumilus,* and *Bacillus sterothermophilus,* and any genetic variants thereof.

Exemplary lactic acid-producing *Lactobacillus* species include, but are not limited to: *Lactobacillus acidophilus, Lactobacillus casei, Lactobacillus DDS-1, Lactobacillus GG, Lactobacillus rhamnosus, Lactobacillus plantarum, Lactobacillus reuteri, Lactobacillus gasserii, Lactobacillus jensenii, Lactobacillus delbruekii, Lactobacillus, bulgaricus, Lactobacillus salivarius* and *Lactobacillus sporogenes* (also designated as *Bacillus coagulans*), and any genetic variants thereof.

Exemplary lactic acid-producing *Sporolactobacillus* species include all *Sporolactobacillus* species, for example, *Sporolactobacillus* P44, and any genetic variants thereof.

Exemplary lactic acid-producing *Bifidiobaclerium* species include, but are not limited to: *Bifidiobacterium adolescentis. Bifidiobacterium animalis, Bifidiobacterium bifidum, Bifidiobacterium hifidus. Bifidiobacterium breve. Bifidiobacterium infantis, Bifidiobacterium infantis. Bijidiobucterium longum,* and any genetic variants thereof.

### 1. Probiotic, Lactic Acid-Producing Bacillus Strains

By way of example, and not of limitation to any particular mechanism, the prophylactic and/or therapeutic effect of a lactic acid-producing bacteria of the present invention results, in part, from a competitive inhibition of the growth of pathogens due to: (*i*) their superior colonization abilities; (*ii*) parasitism of undesirable microorganisms; (*iii*) the production of lactic acid and/or other extracellular products possessing anti-microbial activity; or (*iv*) various combinations thereof. It should be noted that the aforementioned products and activities of the lactic acid-producing *Bacillus* of the present invention act synergistically to produce the beneficial probiotic effect disclosed herein.

A probiotic bacteria which is suitable for use in the methods and compositions of the present invention: (*i*) possesses the ability to produce lactic acid; (*ii*) demonstrates beneficial function; and (*iii*) is non-pathogenic. By way of example and not of limitation, many suitable bacteria have been identified and are described herein, although it should be noted that the present invention is not to be limited to currently-classified bacterial species insofar as the purposes and objectives as disclosed. The physiochemical results from the *in vivo* production of lactic acid is key to the effectiveness of the probiotic lactic acid-producing bacteria of the present invention. Lactic acid production markedly decreases the pH (*i.e.,* increases acidity) within the local micro-floral environment and does not contribute to the growth of many undesirable, physiologically-deleterious bacteria, fungi, and viruses. Thus, the bacterial enzymes and various other metabolic products of the probiotic bacteria (*e.g.*. lactic acid) serve to inhibit the growth of competing pathogenic bacteria, as well as concomitantly increasing the solubility and therefore the bioavailability, of vitamins and minerals within the gastrointestinal tract.

Typical lactic acid-producing bacteria useful as a probiotic of this invention are efficient lactic acid producers which include non-pathogenic members of the *Bacillus* genus which produce bacteriocins or other compounds which inhibit the growth of pathogenic organisms. Exemplary lactic acid-producing, non-pathogenic *Bacillus* species include, but are not limited to: *Bacillus coagulans; Bacillus coagulans Hammer;* and *Bacillus brevis* subspecies *coagulans.*

Several *Bacillus* species which are preferred in the practice of the present invention, include, but are not limited to the lactic acid-producing *Bacillus coagulans* and *Bacillu*s *laevolacticus.* Various other non-lactic acid-producing *Bacillus* species may be utilized in the present invention so long as they produce compounds which possess the ability to inhibit pathogenic bacterial or mycotic growth. Examples of such suitable non-lactic acid-producing *Bacillus* include, but are not limited to: *Bacillus subtilis, Bacillus uniflagellalus. Bacillus lateropsorus, Bacillus laterosporus* BOD. *Bacillus megaterium, Bacillus polymyxa. Bacillus licheniformis, Bacillus pumilus,* and *Bacillus sterothermophilus.*

The *Bacillus* species, particularly those species having the ability to form spores (*e.g., Bacillus coagulans*), are a preferred embodiment of the present invention. The *Bacillus* species utilized in the practice of the present invention may selected from the group comprising: *Bacillus coagulans. Bacillus subtilis, Bacillus laterosporus* and *Bacillus laevolacticus,* all of which have the ability to form spores, and can colonize tissue aerobically. There are a variety of different *Bacillus* species, including, but not limited to many different strains available through commercial and public sources, such as the American Tissue Culture Collection (ATCC). For example, *Bacillus coagulans* strains are available as ATCC Accession Numbers 15949, 8038, 35670, 11369, 23498, 51232, 11014, 31284, 12245, 10545 and 7050. *Bacillus subtilis* strains are available as ATCC Accession Numbers 10783, 15818, 15819, 27505, 13542, 15575, 33234, 9943, 6051a, 25369, 11839, 15811, 27370, 7003, 15563, 4944, 27689, 43223, 55033, 49822, 15561, 15562, 49760, 13933, 29056, 6537, 21359, 21360, 7067, 21394, 15244, 7060, 14593, 9799, 31002, 31003, 31004, 7480, 9858, 13407, 21554, 21555, 27328 and 31524. *Bacillus laterosporus* strains are available as ATCC Accession Numbers 6456, 6457, 30 29653, 9141, 533694, 31932 and 64, including *Bacillus laterosporus BOD. Bacillus laevolacticus* strains are available as ATCC Accession Numbers 23495, 23493, 23494, 23549 and 23492. It should be noted, however, that although many of the examples herein refer to the *Bacillus coagulans* species in particular, it is intended that any of the *Bacillus* species can be used in the compositions, articles of manufacture, systems and method of the present invention.

A *Bacillus* species is particularly suited for the present invention due to the properties in common between species of the *Bacillus* genus, including, but not limited to, the ability to form spores which are relatively resistant to heat and other conditions, making them ideal for storage (shelf-life) in product formulations, and ideal for survival and colonization of tissues under conditions of pH, salinity, and the like on tissues subjected to microbial infection. For example, probiotic *Bacillus coagulans* is non-pathogenic and is generally regarded as safe (*i.e.*, GRAS classification) by the U.S. Federal Drug Administration (FDA) and the U.S. Department of Agriculture (USDA), and by those individuals skilled within the art. Additional useful characteristics of the *Bacillus* species include. but are not limited to: non-pathogenicity, being aerobic, facultative and heterotrophic, thus rendering these species safe, and able to colonize skin, mucous membrane tissues, and various other tissues of inicrest.

Because *Bacillus* species possesses the ability to produce heat-resistant spores, it is particularly useful for making pharmaceutical compositions which require heat and pressure in their manufacture. Accordingly, formulations that include the utilization viable *Bacillus* spores in a pharmaceutically-acceptable carrier are particularly preferred for making and using compositions disclosed in the present invention. The growth of these various *Bacillus* species to form cell cultures, cell pastes, and spore preparations is generally well-known within the art. It should also be noted that the exemplary culture and preparative methods which are described herein for *Bacillus coagulans* may be readily utilized and/or modified for growth and preparation of the other *Bacillus* strains, as well as the lactic acid-producing bacteria disclosed in the present invention. In addition, the exemplary methods and compositions which are described herein using *Bacillus coagulons* as a probiotic for controlling, treating, or reducing microbial infections, may also be readily utilized with other *Bacillus* species.

### 2. Bacillus coagulans

Although, as disclosed herein, any of the aforementioned *Bacillus* strains may be utilized in the practice of the present invention, purified *Bacillus coagulans* is exemplary and preferred as a probiotic for biological control of various microbial pathogens. Because *Bacillus coagulans* forms heat-resistant spores, it is particularly useful for making pharmaceutical compositions for treating microbial infections.

### 2.1 Characteristics and Sources of Bacillus coagulans

Probiotic *Bacillus coagulans* has been categorized as "safe" by the U.S. Federal Drug Administration (FDA), the U.S. Department of Agriculture (USDA), and by those skilled within the art. Examples of its beneficial properties include its ability to reduce the numbers of pathogenic organisms that can kill vitamin producing organisms in the gut, its production of lactase which is a beneficial attribute for those individuals suffering from lactose intolerance, its production of bacteriocins that are inhibitory to those intestinal organisms that produce carcinogens in the colon, and its well known ability to produce L(+) lactic acid, which functions to both metabolize and increase the solubility, absorption, and therefore the bioavailability of vitamins and minerals with the gastrointestinal tract.

The Gram positive rods of *Bacillus congulans* have a cell diameter of greater than 1.0 µm with variable swelling of the sporangium, without parasporal crystal production. *Bacillus coagulans* is a non-pathogenic. Gram positive, spore-forming bacteria that produces L(+) lactic acid (dextrorotatory) under homo-fermentation conditions. It has been isolated from natural sources, such as heat-treated soil samples inoculated into nutrient medium (see *e.g.*, *Bergey's Manual of Systemic Bacteriology,* Vol. 2, Sneath. P.H.A. *et al.,* cds., Williams & Wilkins, Baltimore, MD, 1986). Purified *Bacillus coagulans* strains have served as a source of enzymes including endonucleases (*e.g.*, U.S. Patent No. 5,200,336); amylase (U.S. Patent No. 4,980,180); lactase (U.S. Patent No. 4,323,651) and cyclo-malto-dextrin glucano-transferase (U.S. Patent No. 5,102,800). *Bacillus coagulans* has also been utilized to produce lactic acid (U.S. Patent No. 5,079,164). A strain of *Bacillus coagularrs* (also referred to as *Lactobacillus sporogenes;* Sakaguti & Nakayama, ATCC No. 31284) has been combined with other lactic acid producing bacteria and *Bacillus natto* to produce a fermented food product from steamed soybeans (U.S. Patent No. 4,110,477). *Bacillus coagulans* strains have also been used as animal feeds additives for poultry and livestock to reduce disease and improve feed utilization and, therefore, to increase growth rate in the animals (International PCT Patent Applications No. WO 9314187 and No. WO 9411492). In particular, *Bacillus coagulans* strains have been used as general nutritional materials and agents to control constipation and diarrhea in humans and animals,

Purified *Bacillus coagularts* bacteria utilized in the present invention are available from the American Type Culture Collection (ATCC, Rockville, MD) using the following accession numbers: *Bacillus coagulans* Hammer NRS 727 (ATCC No. 11014); *Bacillus coagulans* Hammer strain C (ATCC No, 11369); *Bacillus coagulans* Hammer (ATCC No. 31284); and *Bacillus coagulans* Hammer NCA 4259 (ATCC No. 15949). Purified *Bacillus coagulans* bacteria are also available from the Deutsche Sarumlung von Mikroorganismen und Zellkuturen GmbH (Braunschweig, Germany) using the following accession numbers: *Bacillus coagulans* Hammer 1915 (DSM No. 2356); *Bacillus coagulans* Hammer 1915 (DSM No. 2383, corresponds to ATCC No. 11014); *Bacillus coagulans* Hammer (DSM No. 2384, corresponds to ATCC No. 11369); and *Bacillus coagulans* Hammer (DSM No. 2385, corresponds to ATCC No. 15949). *Bacillus coagulans* bacteria can also be obtained from commercial suppliers such as Sabinsa Corporation (Piscataway, NJ) or K.K. Fermentation (Kyoto, Japan).

*Bacillus coagulans* strains and their growth requirements have been described previously (see *e.g.,* Baker, D*. et al,* 1960. *Can. J. Microbiol. 6*: 557-563; Nakamura, H. *et al,* 1988. *Int. J. Svst. Bacteriol. 38:* 63-73. In addition, various strains of *Bacillus coagllians* can also be isolated from natural sources (*e.g.,* heat-treated soil samples) using well-known procedures (see *e.g.*, *Bergey's Manual of Systemic Bacteriology Vol. 2*, p. 1117. Sneath.P-H.A. *et al.* eds.. Williams & Wilkins, Baltimore. MD. 1986).

It should be noted that *Bacillus coagulans* had previously been mis-characterized as a *Lactabacillus* in view of the fact that as originally described, this bacterium was labeled as *Lactobacillus sporogenes* (See Nakamura *et al.* 1988. *Int. J. Syst Bacterial. 38*: 63-73). However, initial classification was incorrect due to the fact that *Bacillus coagulans* produces spores and through metabolism excretes L(+)-tactic acid, both aspects which provide key features to its utility. Instead, these developmental and metabolic aspects required that the bacterium be classified as a lactic acid *bacillus,* and therefore it was re-designated. In addition, it is not generally appreciated that classic *Lactobacillus* species are unsuitable for colonization of the gut due to their instability in the harsh (*i.e.,* acidic) pH environment of the bile, particularly human bile. In contrast. *Bacillus coagulans* is able to survive and colonize the gastrointestinal tract in the bile environment and even grown in this low pH range. In particular, the human bile environment is different from the bile environment of animal models, and heretofore there has not been any accurate descriptions of *Bacillus coagulans* growth in human gastrointestinal tract models.

### 2.2 Growth of Bacillus coagulans

*Bacillus coagulans* is aerobic and facultative, grown typically in nutrient broth, pH 5.7 to 6,8, containing up to 2% (wt/vol) NaCl, although neither NaCl nor KCl are an absolute requirement for growth. A pH value ranging from approximately pH 4 to pH 6 is optimum for initiation of sporulation. It is optimally grown at about 30°C to about 55°C, and the spores can withstand pasteurization. It exhibits facultative and heterotrophic growth by utilizing a nitrate or sulfate source.

*Bacillus coagulans* can be grown in a variety of media, although it has been found that certain growth conditions produce a culture which yields a high level of sporulation. For example, sporulation is enhanced if the culture medium includes 10 mg/liter of manganese sulfate, yielding a ratio of spores to vegetative cells of about 80:20. In addition, certain growth conditions produce a bacterial spore which contains a spectrum of metabolic enzymes particularly suited for the present invention (*i.e.,* the control of microbial infections). Although spores produced by these particular growth conditions are preferred, spores produced by any compatible growth conditions are suitable for producing a *Bacillus coagulans* useful in the present invention.

### (i) Culture of Vegetative Bacillus coagulans

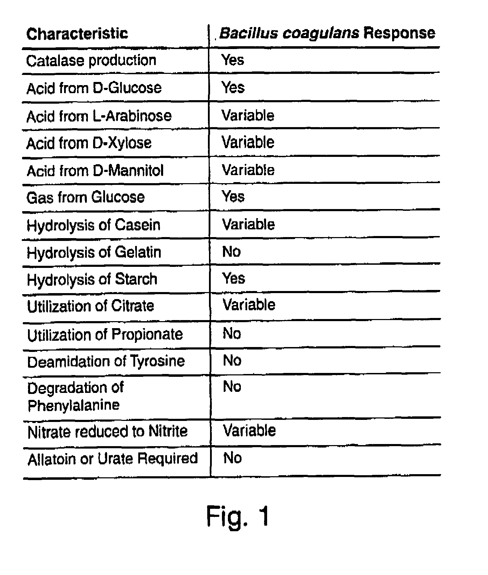

*Bacillus coagulans* is aerobic and facultative, and is typically cultured at pH 5.7 to 6.8. in a nutrient broth containing up to 2% (by wt) NaCl, although neither NaCl, nor KCl are required for growth. A pH of about 4.0 to about 7.5 is optimum for initiation of sporulation (*i.e.,* the formation of spores). The bacteria are optimally grown at 30°C to 45°C, and the spores can withstand pasteurization. Additionally, the bacteria exhibit facultative and heterotrophic growth by utilizing a nitrate or sulfate source. The various metabolic characteristics of *Bacillus coagulans* is illustrated in FIG. 1.

*Bacillus coagulans* can be cultured in a variety of media, although it has been demonstrated that certain growth conditions are more efficacious at producing a culture which yields a high level of sporulation. For example, sporulation is demonstrated to be enhanced if the culture medium includes 10 mg/l of MgSO₄ sulfate, yielding a ratio of spores to vegetative cells of approximately 80:20. In addition, certain culture conditions produce a bacterial spore which contains a spectrum of metabolic enzymes particularly suited for the present invention (*i.e.*, production of lactic acid and enzymes for the enhanced probiotic activity and biodegradation). Although the spores produced by these aforementioned culture conditions are preferred, various other compatible culture conditions which produce viable *Bacillus coagulans* spores may be utilized in the practice of the present invention.

Suitable media for the culture of *Bacillus coagulans* include: PDB (potato dextrose broth); TSB (tryptic soy broth); and NB (nutrient broth), which are all well-known within the field and available from a variety of sources. In one embodiment of the present invention, media supplements which contain enzymatic digests of poultry and/or fish tissue, and containing food yeast are particularly preferred. A preferred supplement produces a media containing at least 60% protein, and about 20% complex carbohydrates and 6% lipids. Media can be obtained from a variety of commercial sources, notably DIFCO (Newark, NJ); BBL (Cockeyesville, MD); Advanced Microbial Systems (Shakopee, MN); and Troy Biologicals (Troy, MD.

In a preferred embodiment of the present invention, a culture of *Bacillus coagulans* Hammer bacteria (ATCC No. 31284) was inoculated and grown to a cell density of approximately 1x10⁸ to 1x10⁹ cells/ml in nutrient broth containing: 5.0 g Peptone; 3.0 g Meat Extract; 10-30 mg MnSO₄ and 1,000 ml distilled water, the broth was then adjusted to pH 7.0. The bacteria were cultured by utilization of a standard airlift fermentation vessel at 30°C. The range of MnSO₄ acceptable for sporulation was found to be 1.0 mg/l to 1.0 g/l. The vegetative bacterial cells can actively reproduce up to 65°C, and the spores are stable up to 90°C.

Following culture, the *Bacillus coagulans* Hammer bacterial cells or spores were collected using standard methods *(e.g.,* filtration, centrifugation) and the collected cells and spores may subsequently be lyophilized, spray dried, air dried or frozen. As described herein, the supernatant from the cell culture can be collected and used as an extracellular agent secreted by *Bacillus coagulans* which possesses anti-microbial activity useful in a formulation of this invention.

A typical yield obtained from the aforementioned culture methodology is in the range of approximately 1x10⁹ to 1x10¹³ viable spores and, more typically, approximately 1x10¹¹ to 1.5x10¹¹ cells/spores per gram prior to being dried. It should also be noted that the *Bacillus coagulans* spores, following a drying step, maintain at least 90% viability for up to 7 years when stored at room temperature. Hence, the effective shelf-life of a composition containing *Bacillus coagulans* Hammer spores at room temperature is approximately 10 years.

### (ii) Preparation of Bacillus coagulans Spores

A culture of dried *Bacillus coagulans* Hammer bacteria (ATCC No. 31284) spores may be prepared as follows. Approximately 1x10⁷ spores were inoculated into one liter of culture medium containing: 24 g (wt./vol.) potato dextrose broth; 10 g of an enzymatic-digest of poultry and fish tissue; 5 g of frueto-oligosaccharides (FOS); and 10 g MnSO₄. The culture was maintained for 72 hours under a high oxygen environment at 37°C so as to produce a culture having approximately 15x10¹⁰ cells/gram of culture. The culture was then filtered to remove the liquid culture medium and the resulting bacterial pellet was resuspended in water and lyophilized. The lyophilized bacteria were ground to a fine "powder" by use of standard good manufacturing practice (GMP) methodologies. The powder is then combined into Formulation 1 or Formulation 4 as described in Specific Example B to form dry powder compositions.

It should also be noted that the most preferred embodiments of the present invention utilizes *Bacillus coagulans* in spore, rather than vegetative bacterial form.

### (iii) Preparation of Bacillus coagulans Extracellular Products

A one liter culture of *Bacillus coagulans* was prepared as described in Section 2(*i*), except that the fructo-oligosaceharide (FOS) was omitted. The culture was maintained for 5 days as described, at which time FOS was added at a concentration of 5 g/liter, and the culture was continued. Subsequently, 20 ml of carrot pulp was then added at day 7, and the culture was harvested when the culture became saturated (*i.e..* no substantial cell division).

The culture was first autoclaved for 30 minutes at 250°F, and then centrifuged at 4000 r.p.m. for 15 mm. The resulting supernatant was collected and filtered in a Buchner funnel through a 0.8 µm filter. The filtrate was collected and further filtered through a 0.2 µm Nalge vacuum filter. The resulting final filtrate was then collected (an approximate volume of 900 ml) to form a liquid containing an extracellular product.

### 3. Bifidogenic Oligosaccharides

Bifidogenic oligosaccharides, as designated herein, are a class of carbohydrates particularly useful for preferentially promoting the growth of a lactic acid-producing bacteria of the present invention. These oligosaccharides include, but are not limited to: fructo-oligosaccharides (FOS); glueo-oligosaccharides (GOS); other long-chain oligosaccharide polymers of fructose and/or glucose; and the trisaccharide - raffinose. All of these aforementioned carbohydrates are not readily digested by pathogenic bacteria. Thus, the preferential growth of lactic acid-producing bacteria is promoted by the utilization of these bifidogenic oligosaccharides due to the nutrient requirements of this class of bacterium, as compared to pathogenic bacteria.

Bifidogenic oligosaccharides are long chain polymers that are utilized almost exclusively by the indigenous *Bifidobacteria* and *Lactobucillus* in the intestinal tract and can be similarly utilized by *Bacillus.* In contrast, physiologically deleterious bacteria such as *Clostridium, Staphylococcus, Salmonella* and *Escherichia coli* cannot metabolize FOS, or other bifidogenic oligosaccharides, and therefor use of these bifidogenic oligosaccharides in combination with a lactic acid-producing bacteria of the present, preferably *Bacillus coagulans,* allows these beneficial, probiotic bacteria to grow and effectively compete with, and eventually replace any undesirable, pathogenic microorganisms within the gastrointestinal tract.

The use of bifidogenic oligosaccharides in the compositions of the present invention provides a synergistic effect thereby increasing the effectiveness of the probiotic-containing compositions disclosed herein. This synergy is manifested by selectively increasing the ability of the probiotic bacterium to grow by, for example, increasing the level of nutrient supplementation which preferentially selects for growth of the probiotic bacteria over many other bacterial species within the infected tissue.

In addition, it is readily understood that *Bifidobacteria* and *Lactobacillus* are also producers of lactic acid. Bifidogenic oligosaccharides enable these aforementioned probiotic organisms to proliferate preferentially over the undesirable bacteria within the gastrointestinal tract, thereby augmenting the probiotic state of the body by further enhancing the solubility of these nutrients (whether of food origin or as a result of nutritional supplement augmentation). Thus, the presence of the bifidogenic oligosaccharides in the compositions of the present invention allows for more effective microbial inhibition by increasing the ability of all varieties of probiotic bacteria to grow, and therefore provide said benefit.

The bifidogenic oligosaccharide of the present invention may be used either alone, or in combination with a lactic acid-producing microorganisms in a therapeutic composition. More specifically, due to the growth promoting activity of bifidogenic oligosaccharides, the present invention contemplates a composition comprising a bifidogenic oligosaccharide present in a concentration sufficient to promote the growth of lactic acid-producing microorganisms. As shown herein, these concentrations amounts can vary widely, as the probiotic microorganisms will respond to any metabolic amount of nutrient oligosaccharide, and therefore the present invention need not be so limited.

A preferred and exemplary bifidogenic oligosaccharide is fructo-oligosaccharide (FOS), although other carbohydrates may also be utilized, either alone or in combination. FOS can be obtained from a variety of natural sources, including commercial suppliers. As a product isolated from natural sources, the components can vary widely and still provide the beneficial agent, namely FOS. Typically, FOS possesses a polymer chain length of from approximately 4 to 200 sugar units, with the longer lengths being preferred. For example, the degree of purity can vary widely so long as biologically-functional FOS is present in the final formulation. Preferred FOS formulations contain at least 50% by weight of fructo-oligosaccharides compared to simple(mono or disaccharide) sugars such as glucose, fructose or sucrose, preferably at least 80% fructo-oligosaccharides (FOS), more preferably at least 90% and most preferably at least 95% FOS. Sugar content and composition can be determined by any of a variety of complex carbohydrate analytical detection methods as is well known. Preferred sources of FOS include, but are not limited to: inulin; Frutafit IQ™ (Imperial Suiker Unie; Sugar Land, Texas); NutraFlora™ (Americal Ingredients, Inc.; Anaheim, CA); and Fruittrimfat Replacers and Sweeteners (Emeryville, CA). Bifidogenic oligosaccharides such as GOS, and other long chain oligosaccharides are also available from commercial vendors.

### 4. Therapeutic Compositions

The various active agents (*i.e*., probiotic bacteria, FOS, and the like) comprising the therapeutic composition of the present invention are combined with a carrier that is physiologically compatible with the gastrointestinal tract tissue of a human or animal to which it is administered. More specifically, the carrier is, preferably, substantially inactive except for surfactant properties which are used in making a suspension of the active ingredients. The therapeutic compositions may also include other physiologically-active constituents which do not interfere with the efficacy of, for example, the *Bacillus coagulans* and FOS as active agents in said composition.

A typical therapeutic composition of the present invention will contain (within a one gram dosage formulation) from approximately 2x10⁷ to 1x10¹⁰ colony forming units (CFU) of viable bacteria or bacterial spores and approximately 10 milligrams (mg) to one gram of fructo-oligosaccharides (FOS).

The therapeutic compositions of the present invention may also include known antioxidants, buffering agents, and other agents such as coloring agents, flavorings, vitamins or minerals. For example, a preferred therapeutic composition may also contain one or more of the following minerals: calcium citrate (15-350 mg); potassium gluconate (5-150 mg); magnesium citrate (5-15 mg); and chromium picollinate (5-200 µg). In addition, a variety of salts may be utilized, including calcium citrate, potassium gluconate, magnesium citrate and chromium picollinate. Thickening agents may be added to the compositions such as polyvinylpyrrolidone, polyethylene glycol or carboxymethylcellulose. Preferred additional components of a therapeutic composition of this invention can include assorted colorings or flavorings, vitamins, fiber, enzymes and other nutrients. Preferred sources of fiber include any of a variety of sources of fiber including, but not limited to: psyllium, rice bran, oat bran, com bran, wheat bran, fruit fiber and the like. Dietary or supplementary enzymes such as lactase, amylase, glucanase, catalase, and the like enzymes can also be included. Chemicals used in the present compositions can be obtained from a variety of commercial sources, including Spectrum Quality Products, Inc (Gardena, CA), Sigma Chemicals (St. Louis, MI), Seltzer Chemicals, Inc., (Carlsbad, CA) and Jarchem Industries, Inc., (Newark, NJ).

The various active agents (*e.g.*, probiotic bacteria, bifidogenic oligosaccharides, and the like) are combined with a carrier which is physiologically compatible with the gastrointestinal tissue of the species to which it is administered. Carriers can be comprised of solid-based, dry materials for formulation into tablet, capsule or powdered form; or the carrier can be comprised of liquid or gel-based materials for formulations into liquid or gel forms. The specific type of carrier, as well as the final formulation depends, in part, upon the selected route(s) of administration.

The therapeutic composition of the present invention may also include a variety of carriers and/or binders. A preferred carrier is micro-crystalline cellulose (MCC), added in alt amount sufficient to complete the one gram dosage total weight. Particularly preferred formulations for a therapeutic composition of this invention are described in the Specific Examples section. Carriers can be solid-based dry materials for formulations in tablet, capsule or powdered form, and can be liquid or gel-based materials for formulations in liquid or gel forms, which forms depend, in part, upon the routes of administration.

Typical carriers for dry formulations include, but are not limited to: trehalose, malto-dextrin, rice flour, micro-crystalline cellulose (MCC) magnesium sterate, inositol, FOS, GOS, dextrose, sucrose, and like carriers. Where the composition is dry and includes evaporated oils that produce a tendency for the composition to cake (adherence of the component spores, salts, powders and oils), it is preferred to include dry fillers which distribute the components and prevent caking. Exemplary anti-caking agents include MCC, talc, diatomaceous earth, amorphous silica and the like, and are typically added in an amount of from approximately 1% to 95% by weight. It should also be noted that dry formulations which are subsequently rehydrated (*e.g.,* liquid formula) or given in the dry state (*e.g.*, chewable wafers, pellets or tablets) are preferred to initially hydrated formulations. Dry formulations (*e.g.*, powders) may be added to supplement commercially available foods (*e.g.*, liquid formulas, strained foods, or drinking water supplies). Similarly, the specific type of formulation depends upon the route of administration.

Suitable liquid or gel-based carriers include but are not limited to: water and physiological salt solutions; urea; alcohols and derivatives (*e.g,* methanol, ethanol, propanol, butanol); glycols (*e.g.,* ethylene glycol, propylene glycol, and the like). Preferably, water-based carriers possess a neutral pH value (*i.e.,* pH 7.0). The compositions may also include natural or synthetic flavorings and food-quality coloring agents, all of which must be compatible with maintaining viability of the lactic acid-producing microorganism. Well-known thickening agents may also be added to the compositions such as com starch, guar gum, xanthan gum, and the like. Where a liquid-based composition containing spores is provided, it is desirable to include a spore germination inhibitor to promote long term storage. Any spore germination inhibitor may be used. By way of example and not of limitation, preferred inhibitors include: hyper-saline carriers, methylparaben, guargum, polysorbates, preservatives, and the like.

Other suitable carriers include, but are not limited to aqueous and oleaginous carriers (*e.g.*, white petrolatum, isopropyl myristate, lanolin or lanolin alcohols, mineral oil, sorbitan mono-oleate, propylene glycol, cetylstearyl alcohol, together or in various combinations); hydroxypropyl cellulose (MW = 100.000 to 1.000.000); detergents (*e.g.*, polyoxyl stearate or sodium lauryl sulfate) and mixed with water to form a gel, liquid or semi-solid composition. Other suitable carriers comprise water-in-oil or oil-in-water emulsions and mixtures of emulsifiers and emollients with solvents such as sucrose stearate, sucrose cocoate, sucrose distcarate, mineral oil, propylene glycol, 2-ethyl-1,3 polyoxypropylene-15-stearyl ether and water. Emulsions containing water, glycerol stearate, glycerin, mineral oil, synthetic spermaceti, cetyl alcohol, butylparaben, propylparaben and methylpaiaben are all commercially available. Preservatives may also be included in the carrier and include, methylparaben, propylparaben, benzyl alcohol and ethylene diamine tetraacetate (EDTA) salts. Well-known flavorings and/or colorants may also be included in the carrier. The composition may also include a plasticizer such as glycerol or polyethylene glycol (MW= 800 to 20,000). It should be noted that, generally, the composition of the carrier can be varied so long as it does not interfere significantly with the pharmacological activity of the active ingredients or the viability of the *Bacillus coagulans* spores.

Preservatives may also be included within the carrier including methylparaben, propylparaben, benzyl alcohol and ethylene diamine tetraacetate salts. Well-known flavorings and/or colorants may also be included within the carrier. The compositions of the present invention may also include a plasticizer such as glycerol or polyethylene glycol (with a preferred molecular weight of MW = 800 to 20,000). The composition of the carrier can be varied so long as it does not interfere significantly with the pharmacological activity of the active ingredients or the viability of the *Bacillus coagulans* spores.

A therapeutic composition of the present invention can be formulated to be suitable for oral administration in a variety of ways, for example in a liquid, a powdered food supplement, a paste, a gel, a solid food, a packaged food, a wafer, and the like as described in more detail in the Examples. Other formulations will be readily apparent to one skilled within the art.

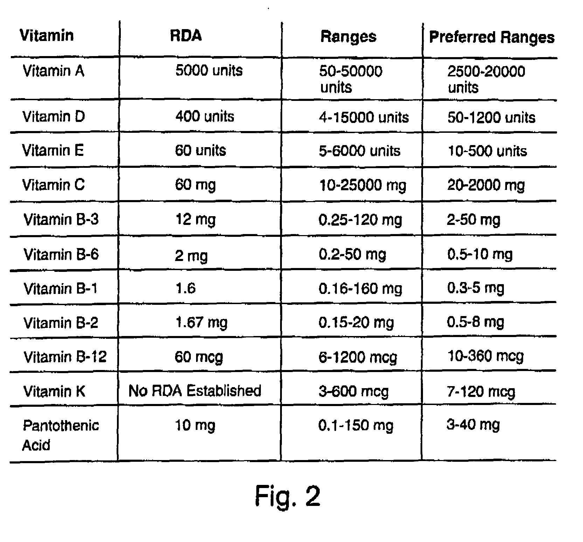

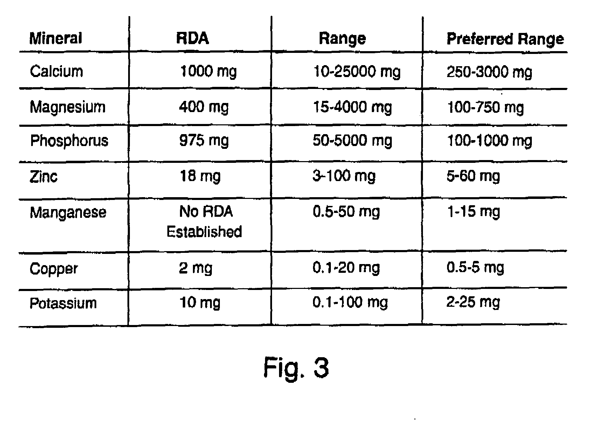

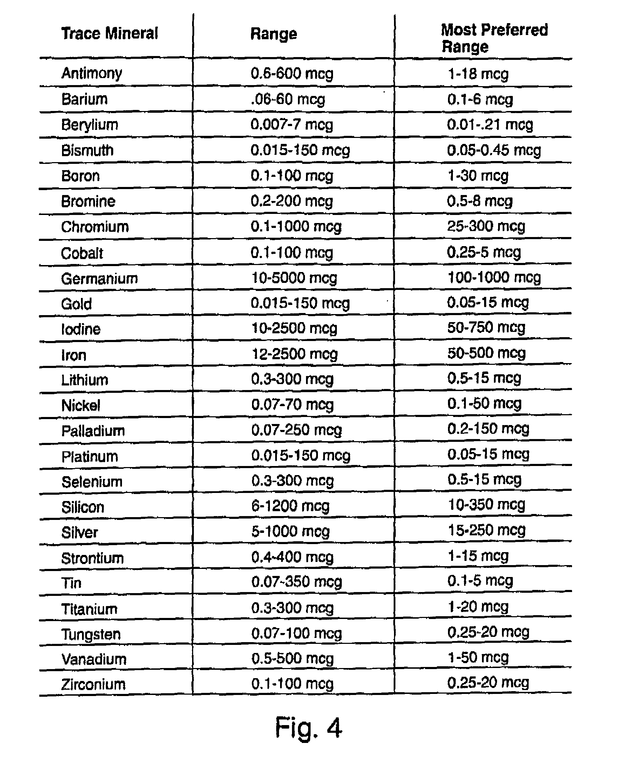

A nutrient supplement component of a composition of this invention can include any of a variety of nutritional agents, as are well known, including vitamins, minerals, essential and non-essential amino acids, carbohydrates, lipids, foodstuffs, dietary supplements, and the like. Preferred compositions comprise vitamins and/or minerals in any combination. Vitamins for use in a composition of this invention can include vitamins B, C, D, E, folic acid, K. niacin, and like vitamins. The composition can contain any or a variety of vitamins as may be deemed useful for a particularly application, and therefore, the vitamin content is not to be construed as limiting. Typical vitamins are those, for example, recommended for daily consumption and in the recommended daily amount (PDA), although precise amounts can vary. FIG. 2 illustrates, in tabular form, the vitamins, their RDA. their ranges and respective preferred ranges, of the therapeutic compositions of the present invention. The composition would preferably include a complex of the RDA vitamins, minerals and trace minerals as well as those nutrients that have no established RDA, but have a beneficial role in healthy human or mammal physiology. The preferred major minerals and their respective RDA are shown in FIG. 3. The preferred mineral format would include those that are in either the gluconate or citrate form because these forms are more readily metabolized by lactic acid bacteria. FiG. 4, illustrates, in tabular form, the preferred trace minerals and their respective ranges, as utilized in the therapeutic composition of the present invention.

In a related embodiment, the invention contemplates a composition comprising a viable lactic acid-producing bacteria in combination with any material to be adsorbed, including but not limited to nutrient supplements, foodstuffs, vitamins, minerals, medicines, therapeutic compositions, antibiotics, hormones, steroids, and the like compounds where it is desirable to insure efficient and healthy absorption of materials from the gastrointestinal track into the blood. The amount of material included in the composition can vary widely depending upon the material and the intended purpose for its absorption, such that the invention is not to be considered as limiting.

A number of methodologies are disclosed herein for increasing the solubility and, therefore, the bioavailability of vitamins and minerals in food and nutritional formulations. The present invention contemplates a method for increasing the bioavailability of vitamins and minerals using lactic acid-producing bacteria, preferably *Bacillus coagularrs* spores. It should be noted however that the scope of the invention is not to be limited to any particular member of the *Bacillus* genera, but can include any member of the *Bacillus, Lactobacillus, Bifidobacterium, Sporolaetobaeillus, Streptococcus,* and/or *Eruerococcus* genus. The aforementioned method comprises administration of a therapeutic composition of the present invention containing the active ingredients, to a human or animal, in various dosage regimens as described herein to achieve the desired nutritional result.

Administration of a nutritional composition is, preferably, to the gastrointestinal tract by use of a gel, suspension, aerosol spray, capsule, tablet, wafer, powder or semi-solid formulation (*e.g.,* a suppository) containing a therapeutic composition of the present invention, all formulated using methods well-known within the art. Administration of the compositions containing the active ingredients effective in producing lactic acid and/or the appropriate enzymes for solublizing vitamins or minerals generally consist of from one to ten unit dosages of 10 milligrams to 10 grams per dosage of said composition for a total time period of from one day to one month. Unit dosages are generally given once every twelve hours, and up to once every four hours. Preferably, one to four dosages of the therapeutic composition is administered per day. Exemplar formulations are provided in the Specific Examples section, *infra.*

### 5. Specific Examples

The following examples relating to the present invention are illustrative, and should not be construed as expressly limiting the invention in any manner. Moreover, such variations of the invention, now known or later developed, which would be within the purview of one skilled within the art are to be considered to fall within the scope of the present invention hereinafter claimed.

### Formulation 1 (Combination Vitamin/ Mineral Supplement)

| | |
|---|---|
| *Bacillus coogulans* | 5x10⁸ viable spores (approximately 35 mg) |
| Calcium citrate | 35 milligrams (mg) |
| Potassium gluconate | 20 mg |
| Magnesium citrate | 20 mg |
| Chromium picollinate | 100 micrograms (µg) |
| Fructo-oligosaccharides (FOS) | 250 mg |
| Vitamin A | 10,000 International Units (IU) |
| Vitamin C | 120 mg |
| Vitamin D | 400 IU |
| Vitamin B-1 | 5 mg |
| Vitamin B-2 | 8 mg |
| Vitamin B-6 | 8 mg |
| Vitamin B-12 | 15 µg |
| Pantothenic Acid | 30 mg |
| Folic Acid | 200 milli-equivalents (meq) |
| Vitamin E | 75 IV |

| | |
|---|---|
| The balance of the 1 gram capsule, tablet, or wafer will be microcrystaline cellulose (MCC) | |

### Formulation 2 (Mineral Supplements)

| | |
|---|---|
| *Bacillus coagulans* | 2.5x10⁸ viable spores (approximately 17.5 mg) |
| Calcium citrate | 35 mg |
| Potassium gluconate | 10 mg |
| Magnesium citrate | 10 mg |
| Chromium picollinate | 50 µg |
| Fructo-oligosaccharides (FOS) | 100 mg |
| Micro-crystalline cellulose (MCC) | 827.5 mg |

### Formulation 3 (Vitamin Supplement)

| | |
|---|---|
| *Bacillus coagularrs* | 2.5x10⁸ viable spores (approximately 17.5 mg) |
| Vitamin A | 10,000 IU |
| VitaminB-1 | 5 mg |
| VitaminB-2 | 8 mg |
| ViMmiaB-6 | 8 mg |
| VitaminB-12 | 15 µg |
| Vitamin E | 75 IU |
| Vitamin C | 120 mg |
| Lactase | 200 IU |
| FOS | 400 mg |

| | |
|---|---|
| The balance of the 1 gram capsule, tablet, or wafer will be microcrystaline cellulose (MCC). | |

### Equivalents

From the foregoing detailed description of the specific embodiments of the present invention, it should be readily apparent that unique, improved methodologies for the utilization of bacterial enzymes and various other metabolic products (*e.g.*, lactic acid) derived from lactic acid producing bacteria, preferably *Bacillus coagulans,* to increase the solubility and, therefore, the bioavailability, of vitamins and minerals within the gastrointestinal tract of animals and humans, has been disclosed. Although particular embodiments have been disclosed herein in detail, this has been done by way of example for purposes of illustration only, and is not intended to be limiting with respect to the scope of the appended claims which follow. In particular, it is contemplated by the inventor that various substitutions, alterations, and modifications may be made to the invention without departing from the spirit and scope of the invention as defined by the claims. For example, the final form (*e.g.*, stabilized gel, cream, emulsification, and the like) which is selected for the therapeutic composition of the present invention is believed to be a matter of routine for a person of ordinary skill in the art with knowledge of the embodiments described herein.

## Claims

1. Non-therapeutic use of one or more non-pathogenic, lactic acid-producing strains of *Bacillus coagulans,* or of an extracellular supernatant derived from a culture of one or more such strains, within a nutritionally-acceptable carrier, which carrier is suitable for administration to the gastrointestinal tract of a vertebrate animal, as a nutritional composition for increasing the solubility and bioavailability of nutritional minerals within the gastrointestinal tract of the animal.

2. Use as claimed in claim 1, wherein said one or more strains of *Bacillus coagulans* are selected from the group consisting of *Bacillus coagulans, Bacillus coagulans Hammer, Bacillus brevis* subspecies *coagulans* and any genetic variants thereof.

3. Use as claimed in claim 1 or claim 2, wherein said nutritional minerals are present in the form of gluconates or citrates.

4. Use as claimed in claim 1 or claim 2, wherein said nutritional composition further includes one or more minerals in gluconate or citrate form.

5. Use according to any preceding claim, wherein said minerals arc selected from the group consisting of calcium, magnesium, phosphorus, zinc, manganese, copper, potassium, antimony, barium, beryllium, bismuth, boron, bromine, chromium, cobalt, germanium, gold, iodine, iron, lithium, nickel, palladium, platinum, selenium, silicon, silver, strontium, tin, titanium, tungsten, vanadium and zirconium.

6. Use according to any preceding claim, wherein said minerals are selected from calcium citrate, potassium gluconate and magnesium citrate.

7. Use according to any preceding claim, wherein said minerals further include chromium picohinate.

8. Use according to any preceding claim, wherein said *Bacillus coagulans* bacterial strain is included in said composition in a form selected from a group consisting of a dried bacterial cell mass contained with a flowable concentrate, a stabilised gel, or a stabilised paste.

9. Use according to any of claims 1-7, wherein said *Bacillus coagulans* bacterial strain is in the form of a dried bacterial spore mass, which spores possess the ability to germinate following administration, contained within a flowable concentrate, a stabilised gel, or a stabilised paste.

10. Use according to any preceding claim, wherein the total administered concentration of said nutritional composition ranges from 10 milligrams to 10 grams of composition per day.

11. Use according to any preceding claim, wherein the total administered concentration of *Bacillus coagulans* within said nutritional composition ranges from 1x10³ to 1x10¹² variable bacteria or spores per day.

12. Use according to any preceding claim, wherein said nutritional composition additionally contains one or more bifidogenic factors.

13. Use according to claim 12, wherein said one or more bifidogenic factors comprise a fructo-oligosaccharide (FOS), and wherein the total administered concentration of the one or more bifidogenic factors within said nutritional composition ranges from 10 milligrams to 20 grams per gram of nutritional composition per day.

14. Use according to any preceding claim, wherein the physiological location of the administration of said nutritional composition is selected from a group consisting of buccal; topical; vaginal; nasal; ocular; and otic administration locations.

15. Use according to any preceding claim, wherein said nutritional composition additionally comprises one or more vitamins selected from the group consisting of Vitamin A, Vitamin D, Vitamin E, Vitamin C, Vitamin B-3, Vitamin B-6, Vitamin B-1, Vitamin B-2, Vitamin B-12, Vitamin K and Pantothenic Acid.

16. Use according to any preceding claim, wherein said nutritional composition additionally comprises one or more vitamins in the following ranges of concentrations:
per gram of concentration:-
| Vitamin | concentration range (per gram) |
|---|---|
| A | 50 - 50,000 IU |
| D | 4 - 15,000 IU |
| E | 5 - 6,000 IU |
| C | 10 - 25,000 mg |
| B-3 | 0.25 - 120 mg |
| B-6 | 0.20 - 50 mg |
| B-1 | 0.16 - 160 mg |
| B-2 | 0.15 - 20 mg |
| B-12 | 6 µg - 1.2 mg |
| K | 3 µg - 600 µg |
| Pantothenic Acid | 0.1 - 150 mg |

17. Use according to any preceding claim, wherein said minerals comprise one or more minerals in the following ranges of concentrations:
in milligrams or micrograms per gram of composition:-
| Mineral | concentration range (per gram) |
|---|---|
| calcium | 10 - 25,000 mg |
| magnesium | 15 - 4,000 mg |
| phosphorous | 50 - 5,000 mg |
| zinc | 3 - 100 mg |
| manganese | 0.5 - 50 mg |
| copper | 0.1 - 20 mg |
| potassium | 0.1 - 100 mg |
| antimony | 0.6 - 600 mg |
| barium | 0.06 - 60 mg |
| beryllium | 0.007 - 7 µg |
| bismuth | 0.015 - 150 µg |
| boron | 0.1 - 100 µg |
| bromine | 0.2 - 200 µg |
| chromium | 0.1 - 1000 µg |
| cobalt | 0.1 - 100 µg |
| germanium | 10 - 5000 µg |
| gold | 0.015 - 150 µg |
| iodine | 10 - 2500 µg |
| iron | 12 - 2500 µg |
| lithium | 0.3 - 300 µg |
| nickel | 0.07 - 70 µg |
| palladium | 0.07 - 250 µg |
| platinum | 0.015 - 150 µg |
| selenium | 0.3 - 300 µg |
| silicon | 6 - 1200 µg |
| silver | 5 - 1000 µg |
| strontium | 0.4 - 400 µg |
| tin | 0.07 - 350 µg |
| titanium | 0.3 - 300 µg |
| tungsten | 0.07 - 100 µg |
| vanadium | 0.5 - 500 µg |
| zirconium | 0.1 - 100 µg |

18. Use according to any preceding claim, wherein said nutritional composition is in the form of a wafer, capsule or tablet.

19. A pharmaceutical or nutritional composition comprising a therapeutically-effective concentration of one or more nan-pathogenic, lactic acid-producing strains of *Bacillus coagulans,* or an extracellular supernatant derived from a culture of one or more such strains or species, within a pharmaceutically or nutritionally-acceptable carrier, which carrier is suitable for administration to the gastrointestinal tract of a vertebrate animal, and nutritional minerals selected from the group consisting of calcium citrate, magnesium citrate and minerals in gluconate form, wherein said lactic acid-producing bacterial species or strain possesses the ability to increase the solubility and bioavailability of said minerals within the gastrointestinal tract of the animal.

20. A composition as claimed in claim 19, wherein said one or more strains of *Bacillus coagulans* are selected from the group consisting of *Bacillus coagulans, Bacillus coagulans Hammer, Bacillus brevis* subspecies *coagulans* and any genetic variants thereof

21. A composition as claimed in claim 19 or claim 20, further comprising chromium picollinate.

22. Use of a non-pathogenic, lactase-producing bacterial species or strain in the manufacture of a composition for administration to the gastrointestinal tract of a vertebrate animal for the treatment of lactose intolerance in said animal.

23. Use according to claim 22, wherein said bacterial species or strain is *Bacillus coagulans.*

24. Use according to claim 23, wherein said *Bacillus coagulans* is selected from *Bacillus coagulans, Bacillus coagulans Hammer* and *Bacillus brevis* subspecies *coagulans* and any genetic variants thereof.

25. Use according to claim 23 or claim 24, wherein said *Bacillus coagulans* is included in said composition as a dried bacterial cell mass contained with a flowable concentrate, a stabilised gel or a stabilised paste.

26. Use according to claim 23 or claim 24, wherein said *Bacillus coagulans* is used in the form of a dried bacterial spore mass, which spore mass possesses the ability to germinate following administration, contained within a flowable concentrate, a stabilised gel or a stabilised paste.

27. Use according to any of claims 23-26, wherein the total concentration of said composition to be administered per day ranges from 10 milligrams to 10 grams of composition.

28. Use according to any of claims 23-27, wherein the total concentration of *Bacillus coagulans* to be administered per day within said composition ranges from 1x10³ to 1x10¹² variable bacteria or spores.

29. Use according to any of claims 23-28, wherein said composition additionally contains one or more bifidogenic factors.

30. Use according to claim 29, wherein said one or more bifidogenic factors comprise a fructo-oligosaccharide, the total concentration of the one or more bifidogenic factors to be administered per day within said composition ranging from 10 milligrams to 20 grams per gram of composition.

31. Use according to any preceding claim, wherein said composition is in the form of a wafer, capsule or tablet.

32. A therapeutic composition comprising a therapeutically-effective concentration of one or more non-pathogenic, lactic acid-producing bacterial species or strains within a pharmaceutically-acceptable carrier suitable for administration to the gastrointestinal tract of a vertebrate, wherein said lactic acid-producing bacterial species or strain possesses the ability to increase the solubility and bioavailability of nutritional materials within the gastrointestinal tract of an animal or, preferably, a human.

33. The therapeutic composition of claim 32, wherein said non-pathogenic, lactic acid-producing bacterial species or strain is a *Bacillus* bacterial species which is selected from a group comprising: *Bacillus coagulans; Bacillus coagulans Hammer; Bacillus brevis* subspecies *coagulans, Bacillus subtilis, Bacillus uniflagellatus, Bacillus lateropsorus, Bacillus laterosporus* BOD, *Bacillus megaterium, Bacillus polymyxa, Bacillus licheniformis, Bacillus pumilus,* and *Bacillus sterothermophilus,* and any genetic variants thereof.

34. The therapeutic composition of claim 32, wherein said non-pathogenic, lactic acid-producing bacterial species or strain is a *Lactobacillus* bacterial species which is selected from a group comprising: *Lactobacillus acidophilus, Lactobacillus casei, Lactobacillus DDS-1, Lactobacillus GG, Lactobacillus rhamnosus, Lactobacillus plantarum, Lactobacillus reuteri, Lactobacillus gasserii, Lactobacillus jensenil, Lactobacillus delbruekii, Lactobacillus, bulgaricus, Lactobacillus salivarius* and *Lactobacillus sporogenes* (also designated as *Bacillus coagulans*), and any genetic variants thereof.

35. The therapeutic composition of claim 32, wherein said non-pathogenic, lactic acid-producing bacterial species or strain is a *Sporolactobacillus* bacterial species which is selected from a group comprising: all *Sporolactobacillus* species, for example, *Sporolactobacillus* P44, and any genetic variants thereof.

36. The therapeutic composition of claim 32, wherein said non-pathogenic, lactic acid-producing bacterial species or strain is a *Rifidiobacterium* bacterial species which is selected from a group comprising: *Bifidiobacterium adolescentis, Bifidiobacterium animalis, Bifidiobacterium bifidum, Bifidiobacterium bifidus, Bifidiobacterium breve, Bifidiobacterium infantis, Bifidiobacterium infantis, Bifidiobacterium longum,* and any genetic variants thereof:

37. A therapeutic composition comprising a therapeutically-effective concentration of one or more strains of the non-pathogenic, lactic acid-producing bacteria *Bacillus coagulans*; wherein said *Bacillus coagulans* bacterial strain are selected from a group comprising *Bacillus coagulans*; *Bacillus coagulans Hammer*; and *Bacillus brevis* subspecies *coagulans,* and any genetic variants thereof, within a pharmaceutically-acceptabl carrier suitable for administration to the gastrointestinal tract of a vertebrate, and wherein said *Bacillus coagulans* strain possesses the ability to increase the solubility and bioavailability of nutritional materials within the gastrointestinal tract of an animal or, preferably, a human.

38. The therapeutic composition of claim 37, wherein said *Bacillus coagulans* bacterial stain is included in said composition in a form selected from a group consisting of a dried bacterial cell mass contained with a flowable concentrate, a stabilized gel, or a stabilized paste.

39. The therapeutic composition of claim 37, wherein said *Bacillus coagulans* bacterial stain is in form of a dried bacterial spore mass, which possess the ability to germinate following administration, contained within a flowable concentrate, a stabilized gel, or a stabilized paste.

40. The therapeutic composition of claim 37, wherein the total administered concentration of said therapeutic composition preferably ranges from approximately 10 milligrams to approximately 10 grams of composition per day, more preferably ranges from approximately 0.1 grams to approximately 5 grams of composition per day, and most preferably ranges from approximately 0.5 grams to approximately 1 gram of composition per day.

41. The therapeutic composition of claim 37, wherein the total administered concentration of *Bacillus coagulans* within said therapeutic composition preferably ranges from approximately 1x10³ to approximately 1x10¹² viable bacteria or spores per day, more preferably ranges from approximately 1x10⁵ to approximately 1x10¹⁰ viable bacteria or spores per day, and most preferably ranges from approximately 2x10⁷ to approximately 1x10¹⁰ viable bacteria or spores per day.

42. The therapeutic composition of claim 37, wherein said therapeutic composition additionally contains one or more bifidogenic factors.

43. The therapeutic composition of claims 37 or 42, wherein said bifidogenic factor is a fructo-oligosaccharide (FOS), and wherein the total administered concentration of the bifidogenic factor within said therapeutic composition ranges preferably ranging from approximately 10 milligrams to approximately 20 grams per gram of therapeutic composition per day, more preferably from approximately 50 milligrams to approximately 10 grams per gram of therapeutic composition per day, and most preferably from approximately from approximately 150 milligrams to approximately 1 gram per gram of therapeutic composition per day.

44. The therapeutic composition of claim 37, wherein the physiological location of the administration of said therapeutic composition is selected from a group comprising: buccal; topical; vaginal; nasal; ocular; and otic administration locations.

45. The therapeutic composition of claim 37, wherein said therapeutic composition additionally comprises one or more vitamins selected from the group comprising: Vitamin A, Vitamin D, Vitamin E, Vitamin C, Vitamin B-3, Vitamin B-6, Vitamin B-1, Vitamin B-2, Vitamin B-12, Vitamin K, and Pantothenic Acid.

46. The therapeutic composition of claims 37 or 45, wherein said therapeutic composition additionally comprises one or more vitamins in the following ranges of concentrations and wherein: the concentration of Vitamin A ranges from approximately 50 IU to approximately 50,000 IU and preferably ranges from approximately 2500 IU to approximately 20,000 IU; the concentration of Vitamin D ranges from approximately 4 IU to approximately 15,000 IU and preferably ranges from approximately 50 IU to approximately 1200 IU; Vitamin E, the concentration of Vitamin E ranges from approximately 5 IU to approximately 6000 IU and preferably ranges from approximately 10 IU to approximately 500 IU; the concentration of Vitamin C ranges from approximately 10 milligrams to approximately 25,000 milligrams and preferably ranges from approximately 20 milligrams to approximately 2000 milligrams; the concentration of Vitamin B-3 ranges from approximately 0.25 milligrams to approximately 120 milligrams and preferably ranges from approximately 2 milligrams to approximately 50 milligrams; the concentration of Vitamin B-6 ranges from approximately 0.20 milligrams to approximately 50 milligrams and preferably ranges from approximately 0.5 milligrams to approximately 10 milligrams; the concentration of Vitamin B-1 ranges from approximately 0.16 milligrams to approximately 160 milligrams and preferably ranges from approximately 0.3 milligrams to approximately 5 milligrams; the concentration of Vitamin B-2 ranges from approximately 0.15 milligrams to approximately 20 milligrams and preferably ranges from approximately 0.5 milligrams to approximately 8 milligrams; the concentration of Vitamin B-12 ranges from approximately 6 micrograms to approximately 1200 micrograms and preferably ranges from approximately 10 micrograms to approximately 360 micrograms; the concentration of Vitamin K ranges from approximately 3 micrograms to approximately 600 micrograms and preferably ranges from approximately 7 micrograms to approximately 120 micrograms; and the concentration of Pantothenie Acid ranges from approximately 0.1 milligrams to approximately 150 milligrams and preferably ranges from approximately 3 milligrams to approximately 40 milligrams.

47. The therapeutic composition of claim 37, wherein said therapeutic composition additionally comprises one or more minerals selected from the group comprising: calcium, magnesium, phosphorus, zinc, manganese, copper, potassium, antimony, barium, berylium, bismuth, boron, bromine, chromium, cobalt, germanium, gold, iodine, iron, lithium, nickel, palladium, platinum, selenium, silicon, silver, strontium, tin, titanium, tungsten, vanadium, and zirconium.

48. The therapeutic composition of claims 37 or 47, wherein said therapeutic composition additionally comprises one or more minerals in the following ranges of concentrations and wherein: the concentration of calcium ranges from approximately 10 milligrams to approximately 25,000 milligrams and preferably ranges from approximately 250 milligrams to approximately 3000 milligrams; the concentration of magnesium ranges from approximately 15 milligrams to approximately 4000 milligrams and preferably ranges from approximately 100 milligrams to approximately 750 milligrams; the concentration of phosphorus ranges from approximately 50 milligrams to approximately 5000 milligrams and preferably ranges from approximately 100 milligrams to approximately 1000 milligrams; the concentration of zinc ranges from approximately 3 milligrams to approximately 100 milligrams and preferably ranges from approximately 5 milligrams to approximately 60 milligrams; the concentration of manganese ranges from approximately 0.5 milligrams to approximately 50 milligrams and preferably ranges from approximately 1 milligram to approximately 15 milligrams; the concentration of copper ranges from approximately 0.1 milligrams to approximately 20 milligrams and preferably ranges from approximately 0.5 milligrams to approximately 5 milligrams; the concentration of potassium ranges from approximately 0.1 milligrams to approximately 100 milligrams and preferably ranges from approximately 2 milligrams to approximately 25 milligrams; the concentration of antimony ranges from approximately 0.6 micrograms to approximately 600 micrograms and preferably ranges from approximately 1 microgram to approximately 18 micrograms; the concentration of barium ranges from approximately 0.06 micrograms to approximately 60 micrograms and preferably ranges from approximately 1 microgram to approximately 6 micrograms; the concentration of beryllium ranges from approximately 0.007 micrograms to approximately 7 micrograms and preferably ranges from approximately 0.001 micrograms to approximately 0.21 micrograms; the concentration of bismuth ranges from approximately 0.015 micrograms to approximately 150 micrograms and preferably ranges from approximately 0.05 micrograms to approximately 0.45 micrograms; the concentration of boron ranges from approximately 0.1 micrograms to approximately 100 micrograms and preferably ranges from approximately 1 microgram to approximately 30 micrograms; the concentration of bromine ranges from approximately 0.2 micrograms to approximately 200 micrograms and preferably ranges from approximately 0.5 micrograms to approximately 8 micrograms; the concentration of chromium ranges from approximately 0.1 micrograms to approximately 1000 micrograms and preferably ranges from approximately 25 micrograms to approximately 300 micrograms; the concentration of cobalt ranges from approximately 0.1 micrograms to approximately 100 micrograms and preferably ranges from approximately 0.25 micrograms to approximately 5 micrograms; the concentration of germanium ranges from approximately 10 micrograms to approximately 5000 micrograms and preferably ranges from approximately 100 micrograms to approximately 1000 micrograms; the concentration of gold ranges from approximately 0.015 micrograms to approximately 150 micrograms and preferably ranges from approximately 0.05 micrograms to approximately 15 micrograms; the concentration of iodine ranges from approximately 10 micrograms to approximately 2500 micrograms and preferably ranges from approximately 50 micrograms to approximately 750 micrograms; the concentration of iron ranges from approximately 12 micrograms to approximately 2500 micrograms and preferably ranges from approximately 50 micrograms to approximately 500 micrograms; the concentration of lithium ranges from approximately 0.3 micrograms to approximately 300 micrograms and preferably ranges from approximately 0.5 micrograms to approximately 15 micrograms; the concentration of nickel ranges from approximately 0.07 micrograms to approximately 70 micrograms and preferably ranges from approximately 0.1 micrograms to approximately 50 micrograms; the concentration of palladium ranges from approximately 0.07 micrograms to approximately 250 micrograms and preferably ranges from approximately 0.2 micrograms to approximately 150 micrograms; the concentration of platinum ranges from approximately 0.01 micrograms to approximately 150 micrograms and preferably ranges from approximately 0.05 micrograms to approximately 15 micrograms; the concentration of selenium ranges from approximately 0.3 micrograms to approximately 300 micrograms and preferably ranges from approximately 0.5 micrograms to approximately 15 micrograms; the concentration of silicon ranges from approximately 6 micrograms to approximately 1200 micrograms and preferably ranges from approximately 10 micrograms to approximately 350 micrograms; the concentration of silver ranges from approximately 5 micrograms to approximately 1000 micrograms and preferably ranges from approximately 15 micrograms to approximately 250 micrograms; the concentration of strontium ranges from approximately 0.4 micrograms to approximately 400 micrograms and preferably ranges from approximately 1 microgram to approximately 15 micrograms; the concentration of tin ranges from approximately 0.07 micrograms to approximately 350 micrograms and preferably ranges from approximately 0.1 micrograms to approximately 5 micrograms; the concentration of titanium ranges from approximately 0.3 micrograms to approximately 300 micrograms and preferably ranges from approximately 1 microgram to approximately 20 micrograms; the concentration of tungsten ranges from approximately 0.07 micrograms to approximately 100 micrograms and preferably ranges from approximately 0.25 micrograms to approximately 20 micrograms; the concentration of vanadium ranges from approximately 0.5 micrograms to approximately 500 micrograms and preferably ranges from approximately 1 microgram to approximately 50 micrograms; and the concentration of zirconium ranges from approximately 0.1 micrograms to approximately 100 micrograms and preferably ranges from approximately 0.25 micrograms to approximately 20 micrograms.

49. The therapeutic composition of claim 37, wherein said therapeutic composition additionally comprises an anti-microbial agent which is selected from the group comprising: antibiotics, anti-fungal agents, anti-viral agents, and anti-yeast agents.

50. A therapeutic composition comprising a therapeutically-effective concentration of the extracellular supernatant derived from a culture of one or more strains of the non-pathogenic, lactic acid-producing bacteria *Bacillus coagulans;* wherein said *Bacillus coagulans* bacterial strain are selected from a group comprising *Bacillus coagulans; Bacillus coagulans Hammer;* and *Bacillus brevis* subspecies *coagulans,* and any genetic variants thereof, within a pharmaceutically-acceptable carrier suitable for administration to the gastrointestinal tract of a vertebrate, and wherein said *Bacillus coagulans* strain possesses the ability to increase the solubility and bioavailability of nutritional materials within the gastrointestinal tract of an animal or, preferably, a human.

51. A method of increasing the solubility and bioavailability of nutritional materials within the gastrointestinal tract of an animal or, preferably, a human, comprising the administration of a therapeutically-effective concentration of one or more non-pathogenic, lactic acid-producing bacterial species or strains within a pharmaceutically-acceptable carrier suitable for administration to the gastrointestinal tract of a vertebrate, wherein said lactic acid-producing bacterial species or strain possesses the ability to increase the solubility and bioavailability of nutritional materials within the gastrointestinal tract of an animal or, preferably, a human.

52. The method of claim 51, wherein said non-pathogenic, lactic acid-producing bacterial species or strain is a *Bacillus* bacterial species which is selected from a group comprising: *Bacillus coagulans; Bacillus coagulans Hammer; Bacillus brevis* subspecies *coagulans, Bacillus subtilis, Bacillus uniflagellatus, Bacillus lateropsorur, Bacillus laterosporus* BOD, *Bacillus megaterium, Bacillus polymyxa, Bacillus licheniformis, Bacillus pumilus,* and *Bacillus sterothermophilus,* and any genetic variants thereof.

53. The method of claim 51, wherein said non-pathogenic, lactic acid-producing bacterial species or strain is a *Lactobacillus* bacterial species which is selected from a group comprising: *Lactobacillus acidophilus, Lactobacillus casei, Lactobacillus DDS-1, Lactobacillus GG, Lactobacillus rhamnosus, Lactobacillus plantarum, Lactobacillus reuteri, Lactobacillus gasserii, Lactobacillus jensenii, Lactobacillus delbruekii, Lactobacillus, bulgaficus, Lactobacillus salivarius* and *Lactobacillus sporogenes* (also designated as *Bacillus coagulans*), and any genetic variants thereof.

54. The method of claim 51, wherein said non-pathogenic, lactic acid-producing bacterial species or strain is a *Sporolactobacillus* bacterial species which is selected from a group comprising: all *Sporolactobacillus* species, for example, *Sporolactobacillus* P44, and any genetic variants thereof.

55. The method of claim 51, wherein said non-pathogenic, lactic acid-producing bacterial species or strain is a *Bifidifidiobacterium* bacterial species which is selected from a group comprising: *Bifidiobacterium adolescentis, Bifidiobacterium animatis, Bifidiobacterium bifidum, Bifidiobacterium bifidus, Bifidiobacterium breve, Bifidiobacterium infantis, Bifidiobacterium infantis, Bifidiobacterium longum,* and any genetic variants thereof.

56. A method of increasing the solubility and bioavailability of nutritional materials within the gastrointestinal tract of an animal or, preferably, a human, comprising the administration of a therapeutically-effective concentration of one or more strains of the non-pathogenic, lactic acid-producing bacteria *Bacillus coagulans*; wherein said *Bacillus coagulans* bacterial strain are selected from a group comprising *Bacillus coagulans*; *Bacillus coagulans Hammer*; and *Bacillus brevis* subspecies *coagulans,* and any genetic variants thereof, within a pharmaceutically-acceptable carrier suitable for administration to the gastrointestinal tract of a vertebrate, and wherein said *Bacillus coagulans* strain possesses the ability to increase the solubility and bioavailability of nutritional materials within the gastrointestinal tract of an animal or, preferably, a human.

57. The method of claim 56, wherein said *Bacilluc coagulans* bacterial stain is included in said composition in a form selected from a group consisting of a dried bacterial cell mass contained with a flowable concentrate, a stabilized gel, or a stabilized paste.

58. The method of claim 56, wherein said *Bacillus coagulans* bacterial stain is in form of a dried bacterial spore mass, which possess the ability to germinate following administration, contained within a flowable concentrate, a stabilized gel, or a stabilized paste.

59. The method of claim 56, wherein the total administered concentration of said therapeutic composition preferably ranges from approximately 10 milligrams to approximately 10 grams of composition per day, more preferably ranges from approximately 0.1 grams to approximately 5 grams of composition per day, and most preferably ranges from approximately 0.5 grams to approximately 1 gram of composition per day.

60. The method of claim 56, wherein the total administered concentration of *Bacillus coagulans* within said therapeutic composition preferably ranges from approximately 1x10³ to approximately 1x10¹² viable bacteria or spores per day, more preferably ranges from approximately 1x10⁵ to approximately 1x10¹⁰ viable bacteria or spores per day, and most preferably ranges from approximately 2x10⁷ to approximately 1x10¹⁰ viable bacteria or spores per day.

61. The method of claim 56, wherein said therapeutic composition additionally contains one or more bifidogenic factors.

62. The method of claims 56 or 61, wherein said bifidogenic factor is a fructo-oligosaccharide (FOS), and wherein the total administered concentration of thc bifidogenic factor within said therapeutic composition ranges preferably ranging from approximately 10 milligrams to approximately 20 grams per gram of therapeutic composition per day, more preferably from approximately 50 milligrams to approximately 10 grams per gram of therapeutic composition per day, and most preferably from approximately from approximately 150 milligrams to approximately 1 gram per gram of therapeutic composition per day.

63. The method of claim 56, wherein the physiological location of the administration of said therapeutic composition is selected from a group comprising: buccal; topical; vaginal; nasal; ocular; and otic administration locations.

64. The method of claim 56, wherein said therapeutic composition additionally comprises one or more vitamins selected from the group comprising: Vitamin A, Vitamin D, Vitamin E, Vitamin C, Vitamin B-3, Vitamin B-6, Vitamin B-1, Vitamin B-2, Vitamin B-12, Vitamin K, and Pantothenic Acid.

65. The method of claims 56 or 64, wherein said therapeutic composition additionally comprises one or more vitamins in the following ranges of concentrations and wherein: the concentration of Vitamin A ranges from approximately 50 IU to approximately 50,000 IU and preferably ranges from approximately 2500 IU to approximately 20,000 IU; the concentration of Vitamin D ranges from approximately 4 IU to approximately 15,000 IU and preferably ranges from approximately 50 IU to approximately 1200 IU; Vitamin E, the concentration of Vitamin E ranges from approximately 5 IU to approximately 6000 IU and preferably ranges from approximately 10 IU to approximately 500 IU; the concentration of Vitamin C ranges from approximately 10 milligrams to approximately 25,000 milligrams and preferably ranges from approximately 20 milligrams to approximately 2000 milligrams; the concentration of Vitamin B-3 ranges from approximately 0.25 milligrams to approximately 120 milligrams and preferably ranges from approximately 2 milligrams to approximately 50 milligrams; the concentration of Vitamin B-6 ranges from approximately 0.20 milligrams to approximately 50 milligrams and preferably ranges from approximately 0.5 milligrams to approximately 10 milligrams; the concentration of Vitamin B-1 ranges from approximately 0.16 milligrams to approximately 160 milligrams and preferably ranges from approximately 0.3 milligrams to approximately 5 milligrams; the concentration of Vitamin B-2 ranges from approximately 0.15 milligrams to approximately 20 milligrams and preferably ranges from approximately 0.5 milligrams to approximately 8 milligrams; the concentration of Vitamin B-12 ranges from approximately 6 micrograms to approximately 1200 micrograms and preferably ranges from approximately 10 micrograms to approximately 360 micrograms; the concentration of Vitamin K ranges from approximately 3 micrograms to approximately 600 micrograms and preferably ranges from approximately 7 micrograms to approximately 120 micrograms; and the concentration of Pantothenic Acid ranges from approximately 0.1 milligrams to approximately 150 milligrams and preferably ranges from approximately 3 milligrams to approximately 40 milligrams.

66. The method of claim 56, wherein said therapeutic composition additionally comprises one or more minerals selected from the group comprising: calcium, magnesium, phosphorus, zinc, manganese, copper, potassium, antimony, barium, beryllium, bismuth, boron, bromine, chromium, cobalt, germanium, gold, iodine, iron, lithium, nickel, palladium, platinum, selenium, silicon, silver, strontium, tin, titanium, tungsten, vanadium, and zirconium.

67. The method of claims 56 or 66, wherein said therapeutic composition additionally comprises one or more minerals in the following ranges of concentrations and wherein: the concentration of calcium ranges from approximately 10 milligrams to approximately 25,000 milligrams and preferably ranges from approximately 250 milligrams to approximately 3000 milligrams; the concentration of magnesium ranges from approximately 15 milligrams to approximately 4000 milligrams and preferably ranges from approximately 100 milligrams to approximately 750 milligrams; the concentration of phosphorus ranges from approximately 50 milligrams to approximately 5000 milligrams and preferably ranges from approximately 100 milligrams to approximately 1000 milligrams; the concentration of zinc ranges from approximately 3 milligrams to approximately 100 milligrams and preferably ranges from approximately 5 milligrams to approximately 60 milligrams; the concentration of manganese ranges from approximately 0.5 milligrams to approximately 50 milligrams and preferably ranges from approximately 1 milligram to approximately 15 milligrams; the concentration of copper ranges from approximately 0.1 milligrams to approximately 20 milligrams and preferably ranges from approximately 0.5 milligrams to approximately 5 milligrams; the concentration of potassium ranges from approximately 0.1 milligrams to approximately 100 milligrams and preferably ranges from approximately 2 milligrams to approximately 25 milligrams; the concentration of antimony ranges from approximately 0.6 micrograms to approximately 600 micrograms and preferably ranges from approximately 1 microgram to approximately 18 micrograms; the concentration of barium ranges from approximately 0.06 micrograms to approximately 60 micrograms and preferably ranges from approximately 1 microgram to approximately 6 micrograms; the concentration of beryllium ranges from approximately 0.007 micrograms to approximately 7 micrograms and preferably ranges from approximately 0.001 micrograms to approximately 0.21 micrograms; the concentration of bismuth ranges from approximately 0.015 micrograms to approximately 150 micrograms and preferably ranges from approximately 0.05 micrograms to approximately 0.45 micrograms; the concentration of boron ranges from approximately 0.1 micrograms to approximately 100 micrograms and preferably ranges from approximately 1 microgram to approximately 30 micrograms; the concentration of bromine ranges from approximately 0.2 micrograms to approximately 200 micrograms and preferably ranges from approximately 0.5 micrograms to approximately 8 micrograms; the concentration of chromium ranges from approximately 0.1 micrograms to approximately 1000 micrograms and preferably ranges from approximately 25 micrograms to approximately 300 micrograms; the concentration of cobalt ranges from approximately 0.1 micrograms to approximately 100 micrograms and preferably ranges from approximately 0.25 micrograms to approximately 5 micrograms; the concentration of germanium ranges from approximately 10 micrograms to approximately 5000 micrograms and preferably ranges from approximately 100 micrograms to approximately 1000 micrograms; the concentration of gold ranges from approximately 0.015 micrograms to approximately 150 micrograms and preferably ranges from approximately 0.05 micrograms to approximately 15 micrograms; the concentration of iodine ranges from approximately 10 micrograms to approximately 2500 micrograms and preferably ranges from approximately 50 micrograms to approximately 750 micrograms; the concentration of iron ranges from approximately 12 micrograms to approximately 2500 micrograms and preferably ranges from approximately 50 micrograms to approximately 500 micrograms; the concentration of lithium ranges from approximately 0.3 micrograms to approximately 300 micrograms and preferably ranges from approximately 0.5 micrograms to approximately 15 micrograms; the concentration of nickel ranges from approximately 0.07 micrograms to approximately 70 micrograms and preferably ranges from approximately 0.1 micrograms to approximately 50 micrograms; the concentration of palladium ranges from approximately 0.07 micrograms to approximately 250 micrograms and preferably ranges from approximately 0.2 micrograms to approximately 150 micrograms; the concentration of platinum ranges from approximately 0.015 micrograms to approximately 150 micrograms and preferably ranges from approximately 0.05 micrograms to approximately 15 micrograms; the concentration of selenium ranges from approximately 0.3 micrograms to approximately 300 micrograms and preferably ranges from approximately 0.5 micrograms to approximately 15 micrograms; the concentration of silicon ranges from approximately 6 micrograms to approximately 1200 micrograms and preferably ranges from approximately 10 micrograms to approximately 350 micrograms; the concentration of silver ranges from approximately 5 micrograms to approximately 1000 micrograms and preferably ranges from approximately 15 micrograms to approximately 250 micrograms; the concentration of strontium ranges from approximately 0.4 micrograms to approximately 400 micrograms and preferably ranges from approximately 1 microgram to approximately 15 micrograms; the concentration of tin ranges from approximately 0.07 micrograms to approximately 350 micrograms and preferably ranges from approximately 0.1 micrograms to approximately 5 micrograms; the concentration of titanium ranges from approximately 0.3 micrograms to approximately 300 micrograms and preferably ranges from approximately 1 microgram to approximately 20 micrograms; the concentration of tungsten ranges from approximately 0.07 micrograms to approximately 100 micrograms and preferably ranges from approximately 0.25 micrograms to approximately 20 micrograms; the concentration of vanadium ranges from approximately 0.5 micrograms to approximately 500 micrograms and preferably ranges from approximately 1 microgram to approximately 50 micrograms; and the concentration of zirconium ranges from approximately 0.1 micrograms to approximately 100 micrograms and preferably ranges from approximately 0.25 micrograms to approximately 20 micrograms.

68. The method of claim 56, wherein said therapeutic composition additionally comprises an anti-microbial agent which is selected from the group comprising: antibiotics, anti-fungal agents, anti-viral agents, and anti-yeast agents.

69. A method of increasing the solubility and bioavailability of nutritional materials within the gastrointestinal tract of an animal or, preferably, a human, comprising the administration of a therapeutically-effective concentration of the extracellular supernatant derived from a culture of one or more strains of the non-pathogenic, lactic acid-producing bacteria *Bacillus coagulans*; wherein said *Bacillus coagulans* bacterial strain are selected from a group comprising *Bacillus coagulans*; *Bacillus coagulans Hammer;* and *Bacillus brevis* subspecies *coagulans,* and any genetic variants thereof, within a pharmaceutically-acceptable carrier suitable for administration to the gastrointestinal tract of a vertebrate, and wherein said *Bacillus coagulans* strain possesses the ability to increase the solubility and bioavailability of nutritional materials within the gastrointestinal tract of an animal or, preferably, a human.

70. A therapeutic composition comprising one or more *Bacillus* bacterial species or strains within a pharmaceutically-acceptable carrier suitable for oral administration to the gastrointestinal tract of a vertebrate, wherein said *Bacillus coagulans* bacterial strain is capable of growing at a temperature of approximately 30°C to approximately 65°C, produces L(+) dextrorotatory lactic acid, produces spores resistant to temperatures of up to approximately 90°C, and probiotic exhibits activity which increases the solubility and bioavailability of nutritional materials within the gastrointestinal tract of animals or, preferably, humans.

71. The therapeutic composition of claim 70, wherein said probiotic activity results from the vegetative growth of the *Bacillus* bacterial species or strains.

72. The therapeutic composition of claim 70, wherein said probiotic activity results from an extracellular product produced by the isolated *Bacillus* bacterial species or strains.

73. A method of increasing the solubility and bioavailability of vitamins and minerals within the gastrointestinal tract of an animal or, preferably, a human, comprising the administration of a therapeutically-effective concentration of one or more *Bacillus* bacterial species or strains within a pharmaceutically-acceptable carrier suitable for oral administration to the gastrointestinal tract of a vertebrate, wherein said *Bacillus coagulans* bacterial strain is capable of growing at a temperature of approximately 30°C to approximately 65°C, produces L(+) dextrorotatory lactic acid, produces spores resistant to temperatures of up to approximately 90°C, and exhibits probiotic activity which increases the solubility and bioavailability of nutritional materials within the gastrointestinal tract of animals or, preferably, humans.

74. The method of claim 73, wherein said probiotic activity results from the vegetative growth of the *Bacillus* bacterial species or strains.

75. The method of claim 73, wherein said probiotic activity results from an extracellular product produced by the isolated *Bacillus* bacterial species or strains.
